# EUROPEAN PATENT APPLICATION

(11) **EP 4 105 652 A1**
(43) Date of publication of application: **21.12.2022**
(21) Application number: 21754344.6
(22) Date of filing: 08.01.2021
(51) Int. Cl.: G01N 27/27, G01N 27/28, G01N 27/38, G01N 27/416

(54) **WATER QUALITY MEASUREMENT DEVICE AND STORAGE METHOD FOR WATER QUALITY MASUREMENT DEVICE**

(30) Priority: 12.02.2020 JP 2020021286
(71) Applicant: NGK SPARK PLUG CO., LTD., Mizuho-ku Nagoya-shi Aichi 467-8525 (JP)
(72) Inventor: KAMEI, Shunsuke, Nagoya-shi, Aichi 467-8525 (JP); TASHIMA, Keisuke, Nagoya-shi, Aichi 467-8525 (JP); OSAWA, Norimasa, Nagoya-shi, Aichi 467-8525 (JP)
(74) Representative: Grünecker Patent- und Rechtsanwälte PartG mbB
(86) International application number: PCT/JP2021/000417
(87) International publication number: WO 2021/161694

(57) **Abstract**

A water quality measurement device (100) includes a flow channel (130), an ion sensor having a responsive film portion whose surface is disposed in the flow channel (130), a glass-electrode-type sensor having a glass electrode whose surface is disposed in the flow channel (130), a drive section (115), a first opening-closing section, and a second opening-closing section. When a storage condition is satisfied, the water quality measurement device (100) puts the first opening-closing section in a first closed state and puts the second opening-closing section in a second closed state, thereby producing a state in which the surface of the responsive film portion and the surface of the glass electrode are present in a common closed space (135) whereby the closed space (135) is put in a wet state without the surface of the responsive film portion being immersed in the liquid.

## Description

### TECHNICAL FIELD

The present invention relates to a water quality measurement device and to a storage method for a water quality measurement device.

### BACKGROUND ART

Patent Literature 1 discloses an ion sensor including a liquid-membrane-type ion selective electrode. This ion sensor includes a magnesium ion selective electrode that responds selectively to magnesium ion and a calcium ion selective electrode that responds selectively to calcium ion.

Meanwhile, Patent literature 2 discloses a pH sensor including a reference electrode and an acting electrode composed of a glass electrode. This pH sensor has a function of measuring the pH of a liquid under measurement on the basis of outputs of the reference electrode and the acting electrode.

### CITATION LIST

### PATENT LITERATURES

Patent Literature 1: Japanese Patent Application Laid-Open (kokai) No. H7-253410
Patent Literature 2: Japanese Patent Application Laid-Open (kokai) No. 2012-163531

### SUMMARY OF INVENTION

### TECHNICAL PROBLEM

It is useful that a water quality inspection device can measure the concentration of a target ion contained in a liquid under measurement (hereinafter referred to as "measurement target liquid") and the pH of the measurement target liquid in a common path. However, since a storage state suitable for a liquid-membrane-type ion sensor and a storage state suitable for a sensor having a glass electrode differ from each other, storing the two sensors in the common path while allowing them to coexist is difficult.

For example, the liquid-membrane-type ion sensor has a responsive film portion (electrode film that is a liquid membrane portion) containing an ionophore capable of interacting selectively with a particular ion. A sensor of this type cannot be stored with its responsive film portion continuously immersed in a liquid, because continuous immersion of the responsive film portion in the liquid leads to dissolving out of the ionophore.

Meanwhile, the pH sensor that performs pH concentration detection using a glass electrode exhibits a pH concentration measurement function in a state in which a hydrated gel layer exists on the surface of the glass electrode. When the hydrated gel layer is no longer present on the surface of the glass electrode, a separate treatment must be performed so as to recover the pH concentration measurement function. Accordingly, it is desirable for a sensor of such a type to continuously maintain an appropriate amount of moisture so as to prevent drying up of the surface of the glass electrode. This problem may occur not only in pH sensors but in other sensors using glass electrodes.

The present invention has been accomplished so as to solve at least part of the above-described problem and its object is to provide a water quality measurement device which includes an ion sensor having a responsive film portion whose surface is disposed in a flow channel and a sensor using a glass electrode and which can store the two sensors well while allowing the two sensors to coexist in the same device.

### SOLUTION TO PROBLEM

A water quality measurement device, which is a first solution of the present invention, is a water quality measurement device having a flow channel through which a measurement target liquid is forced to flow, including an ion sensor which has a responsive film portion and in which a surface of the above-described responsive film portion is disposed in the above-described flow channel, and measuring the quality of the above-described measurement target liquid. This water quality measurement device comprises: a glass-electrode-type sensor which has a glass electrode and in which a surface of the above-described glass electrode is disposed in the above-described flow channel; a drive section for controlling a flow of a fluid in the above-described flow channel; a first opening-closing section which is provided at a first position and is switched between a first open state for opening the above-described flow channel and a first closed state for closing the above-described flow channel, the first position being located on an upstream side of the above-described ion sensor and the above-described glass-electrode-type sensor in a direction in which the above-described measurement target liquid is forced to flow; a second opening-closing section which is provided at a second position and is switched between a second open state for opening the above-described flow channel and a second closed state for closing the above-described flow channel, the second position being located on a downstream side of the above-described ion sensor and the above-described glass-electrode-type sensor in the direction in which the above-described measurement target liquid is forced to flow; and a supply section for supplying a liquid to the above-described flow channel. When a storage condition is satisfied, this water quality measurement device switches the above-described first opening-closing section to the above-described first closed state and the above-described second opening-closing section to the above-described second closed state, thereby producing a state in which the surface of the above-described responsive film portion and the surface of the above-described glass electrode are present in a common closed space, whereby the above-described closed space is put in a wet state without the surface of the above-described responsive film portion being immersed in the liquid.

When the storage condition is satisfied, the above-described water quality measurement device can produce a state in which the surface of the responsive film portion and the surface of the glass electrode are present in a common closed space, whereby the interior of this closed space can be put in a wet state in which the surface of the responsive film portion is not immersed in liquid. Since this water quality measurement device can store the sensors while maintaining the state in which the surface of the responsive film portion is not immersed in liquid, it is possible to prevent malfunctions that would otherwise occur when the sensors are stored with the responsive film portion continuously immersed in liquid. Also, since this water quality measurement device can store the sensors while maintaining the state in which liquid is present in the closed space, a drop in humidity in the closed space can be suppressed during the storage, whereby drying of the surface of the glass electrode during the storage can be prevented.

The water quality measurement device, which is the first solution of the present invention, may comprise a control section for controlling operations of the above-described drive section, the above-described first opening-closing section, the above-described second opening-closing section, and the above-described supply section. When the above-described storage condition is satisfied, the above-described control section may cause the above-described drive section to perform an operation of producing the above-described wet state.

This water quality measurement device can automatically perform the operation of producing the above-described wet state through own control when the above-described storage condition is satisfied.

In the water quality measurement device, which is the first solution of the present invention, the above-described first opening-closing section may be a first electromagnetic valve for opening and closing the above-described flow channel on the above-described upstream side of the above-described closed space. Similarly, the second opening-closing section may be a second electromagnetic valve for opening and closing the above-described flow channel on the above-described downstream side of the above-described closed space.

By using the electromagnetic valves, this water quality measurement device can reliably open and close the above-described flow channel, at desired timings, at a first position on the upstream side of the above-described closed space and a second position on the downstream side of the above-described closed space. Therefore, during the storage period after the opposite ends have been closed in the above-described wet state, the above-described closed space is more likely to be maintained in a state similar to the state before the closure.

In the water quality measurement device, which is the first solution of the present invention, when the above-described storage condition is satisfied, the above-described control section may cause the above-described drive section to perform an operation of draining the liquid from the above-described closed space and an operation of feeding air into the above-described closed space.

In this water quality measurement device, during the storage period after the opposite ends have been closed in the above-described wet state, the surface of the responsive film portion is more likely to be maintained in a state in which the surface is exposed to air within the closed space. Therefore, this water quality measurement device can reliably prevent malfunctions that would otherwise occur when the sensors are stored with the responsive film portion continuously immersed in liquid. Also, this water quality measurement device can easily produce the wet state (the state in which the surface of the responsive film portion is not immersed in liquid) through drainage of liquid from the closed space and introduction of air into the closed space.

The water quality measurement device, which is the first solution of the present invention, may comprise a flow-rate sensor which can detect the flow rate of the liquid flowing through the above-described closed space. The above-described wet state may be a state in which the flow rate in the above-described closed space detected by the above-described flow-rate sensor has become less than a predetermined value when the above-described drive section is performing the operation of draining the liquid from the above-described closed space and the operation of feeding air into the above-described closed space.

This water quality measurement device can quantitatively determine, on the basis of the "flow rate in the closed space," whether or not the state in which the surface of the responsive film portion is not immersed in liquid has been produced.

In the water quality measurement device, which is the first solution of the present invention, the above-described supply section may include a cleaning section which performs a cleaning operation of feeding cleaning water to the above-described closed space. When the above-described storage condition is satisfied, the above-described control section may cause the above-described cleaning section to perform the cleaning operation and cause the drive section to perform the operation of producing the above-described wet state after the cleaning operation.

When the above-described storage condition is satisfied, this water quality measurement device can perform the operation of cleaning the above-described closed space with the cleaning water and close the opposite ends of the above-described closed space in the above-described wet state after such cleaning operation. Therefore, during the storage period after the opposite ends of the above-described closed space have been closed, this water quality measurement device can store well the ion sensor having a responsive film portion whose surface is disposed in the flow channel and the sensor using a glass electrode, while maintaining the interior of the above-described closed space in a cleaner state.

A storage method for a water quality measurement device, which is a second solution of the present invention, is a storage method for a water quality measurement device having a flow channel through which a measurement target liquid is forced to flow, including an ion sensor which has a responsive film portion and in which a surface of the above-described responsive film portion is disposed in the above-described flow channel, and measuring the quality of the above-described measurement target liquid. The above-described water quality measurement device comprises: a glass-electrode-type sensor which has a glass electrode and in which a surface of the above-described glass electrode is disposed in the above-described flow channel; a drive section for controlling a flow of a fluid in the above-described flow channel; a first opening-closing section which is provided at a first position and is switched between a first open state for opening the above-described flow channel and a first closed state for closing the above-described flow channel, the first position being located on an upstream side of the above-described ion sensor and the above-described glass-electrode-type sensor in a direction in which the above-described measurement target liquid is forced to flow; a second opening-closing section which is provided at a second position and is switched between a second open state for opening the above-described flow channel and a second closed state for closing the above-described flow channel, the second position being located on a downstream side of the above-described ion sensor and the above-described glass-electrode-type sensor in the direction in which the above-described measurement target liquid is forced to flow; and a supply section for supplying a liquid to the above-described flow channel. When a storage condition is satisfied, the above-described storage method switches the above-described first opening-closing section to the above-described first closed state and the above-described second opening-closing section to the above-described second closed state, thereby producing a state in which the surface of the above-described responsive film portion and the surface of the above-described glass electrode are present in a common closed space, whereby the above-described closed space is put in a wet state without the surface of the above-described responsive film portion being immersed in liquid.

When the storage condition is satisfied, the above-described storage method can produce a state in which the surface of the responsive film portion and the surface of the glass electrode are present in a common closed space, whereby the interior of this closed space can be put in a wet state in which the surface of the responsive film portion is not immersed in liquid. Since this storage method can store the sensors while maintaining the state in which the surface of the responsive film portion is not immersed in liquid, it is possible to prevent malfunctions that would otherwise occur when the sensors are stored with the responsive film portion continuously immersed in liquid. Also, since this storage method can store the sensors while maintaining the state in which liquid is present in the above-described closed space, a drop in humidity in the above-described closed space can be suppressed during the storage, whereby drying of the surface of the glass electrode during the storage can be prevented.

In the storage method for a water quality measurement device, which is the second solution of the present invention, the above-described water quality measurement device may comprise a control section for controlling operations of the above-described drive section, the above-described first opening-closing section, the above-described second opening-closing section, and the above-described supply section. When the above-described storage condition is satisfied, the above-described control section may cause the above-described drive section to perform an operation of producing the above-described wet state.

In this storage method, the water quality measurement device can automatically perform the operation of producing the above-described wet state through own control when the above-described storage condition is satisfied.

In the storage method for a water quality measurement device, which is the second solution of the present invention, the above-described first opening-closing section may be a first electromagnetic valve for opening and closing the above-described flow channel on the above-described upstream side of the above-described closed space. Similarly, the second opening-closing section may be a second electromagnetic valve for opening and closing the above-described flow channel on the above-described downstream side of the above-described closed space.

This storage method can reliably open and close the above-described flow channel, at desired timings, at a first position on the upstream side of the above-described closed space and a second position on the downstream side of the above-described closed space by using the electromagnetic valves. Therefore, during the storage period after the opposite ends have been closed in the above-described wet state, the above-described closed space is more likely to be maintained in a state similar to the state before the closure.

In the storage method for a water quality measurement device, which is the second solution of the present invention, when the above-described storage condition is satisfied, the above-described control section may cause the above-described drive section to perform an operation of draining the liquid from the above-described closed space and an operation of feeding air into the above-described closed space.

In this storage method, during the storage period after the opposite ends have been closed in the above-described wet state, the surface of the responsive film portion is maintained in a state in which the surface is exposed to air within the above-described closed space. Therefore, this storage method can reliably prevent malfunctions that would otherwise occur when the sensors are stored with the responsive film portion continuously immersed in liquid. Also, this storage method can easily produce the wet state (the state in which the surface of the responsive film portion is not immersed in liquid) through drainage of liquid from the above-described closed space and introduction of air into the above-described closed space.

In the storage method for a water quality measurement device, which is the second solution of the present invention, the above-described water quality measurement device may comprise a flow-rate sensor which can detect the flow rate of the liquid flowing through the above-described closed space. The above-described wet state may be a state in which the flow rate in the above-described closed space detected by the above-described flow-rate sensor has become less than a predetermined value when the above-described drive section is performing the operation of draining the liquid from the above-described closed space and the operation of feeding air into the above-described closed space.

This storage method can quantitatively determine, on the basis of the "flow rate in the closed space," whether or not the state in which the surface of the responsive film portion is not immersed in liquid has been produced.

In the storage method for a water quality measurement device, which is the second solution of the present invention, the above-described supply section may include a cleaning section which performs a cleaning operation of feeding cleaning water to the above-described closed space. When the above-described storage condition is satisfied, the above-described control section may cause the above-described cleaning section to perform the above-described cleaning operation and cause the above-described drive section to perform the operation of producing the above-described wet state after the above-described cleaning operation.

When the above-described storage condition is satisfied, this storage method can perform the operation of cleaning the above-described closed space with the cleaning water and close the opposite ends of the above-described closed space in the above-described wet state after such cleaning operation. Therefore, during the storage period after the opposite ends of the above-described closed space have been closed, this storage method can store well the ion sensor having a responsive film portion whose surface is disposed in the flow channel and the sensor using a glass electrode, while maintaining the interior of the above-described closed space in a cleaner state.

### ADVANTAGEOUS EFFECTS OF INVENTION

The present invention can store well an ion sensor having a responsive film portion whose surface is disposed in a flow channel and a sensor using a glass electrode while coexisting in the same device.

### BRIEF DESCRIPTION OF DRAWINGS

[FIG. 1] FIG. 1 is a block diagram which conceptually illustrates a water quality measurement device of a first embodiment.
[FIG. 2] FIG. 2 is an explanatory view used for describing a specific example of a first sensor section in the water quality measurement device of FIG. 1.
[FIG. 3] FIG. 3 is an explanatory view used for describing a specific example of a pH sensor in the first sensor section of FIG. 2.
[FIG. 4] FIG. 4 is an explanatory view used for describing a specific example of a liquid-membrane-type ion sensor in the first sensor section of FIG. 2.
[FIG. 5] FIG. 5 is an explanatory view used for describing a specific example of a second sensor section in the water quality measurement device of FIG. 1.
[FIG. 6] FIG. 6 is a flowchart for illustrating the flow of measurement control performed in the water quality measurement device of FIG. 1.
[FIG. 7] FIG. 7 is a flowchart for illustrating the flow of cleaning control performed in the water quality measurement device of FIG. 1.
[FIG. 8] FIG. 8 is a flowchart for illustrating the flow of cancellation control performed in the water quality measurement device of FIG. 1.

### DESCRIPTION OF EMBODIMENTS

### <First embodiment

### 1. Configuration of water quality measurement device

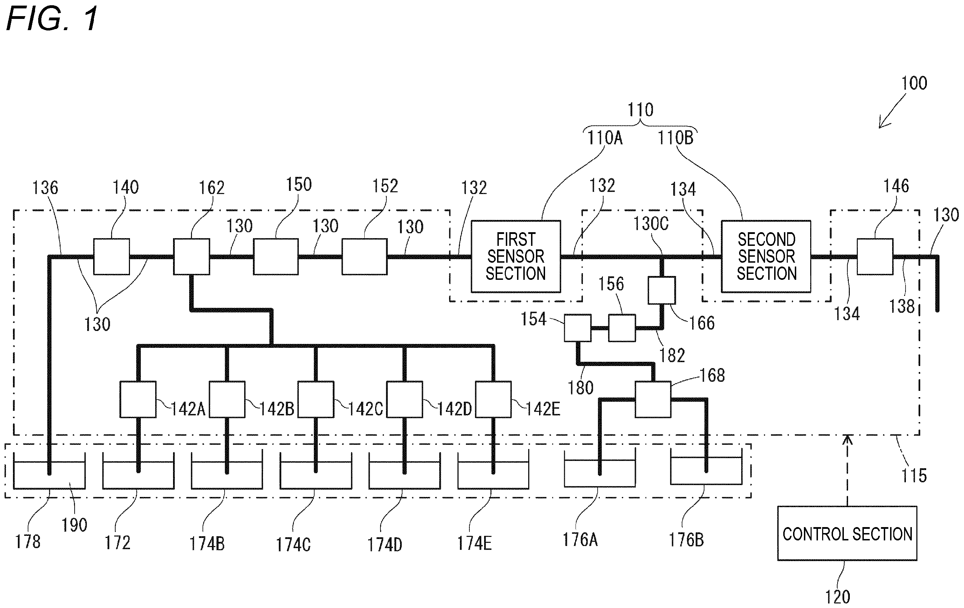

A water quality measurement device 100 according to a first embodiment is illustrated in FIG. 1.

The water quality measurement device 100 of FIG. 1 is a device for measuring the quality of a measurement target liquid. The water quality measurement device 100 may be a device for measuring the quality of breeding water for breeding a creature (for example, breeding water used in the field of aquaculture) as a liquid whose quality is to be measured (hereinafter referred to as "target liquid") or a device for measuring the quality of a target liquid of a type different from breeding water. The water quality measurement device 100 may be one used for water quality management in an aquaculture facility, an aquarium, a hydroponic culture facility, or the like, one used for water quality management in other facilities, or one used for water quality management outside the facilities. The measurement target liquid 190 shown in FIG. 1 may be, for example, water containing salt content (for example, seawater, brackish water, artificial seawater, or the like), water containing no salt content, or water which is low in salinity.

The water quality measurement device 100 is a device for measuring the concentrations of target ions contained in the measurement target liquid. In the example of FIG. 1, the measurement target liquid 190 can be stored in a containing section 178 automatically or manually. The water quality measurement device 100 supplies the measurement target liquid 190 contained in the containing section 178 to a first sensor section 110A and a second sensor section 110B and measures the concentrations of ions contained in the measurement target liquid 190.

The water quality measurement device 100 includes mainly a sensor section 110, a drive section 115, a control section 120, a flow channel 130, a containing section 170, etc.

The containing section 170 has a plurality of containing sections 172, 174B, 174C, 174D, 174E, 176A, 176B, and 178. The containing section 178 is configured to serve as a sampling container for containing the measurement target liquid 190. Notably, in the example of FIG. 1, a single sampling container (containing section 178) is illustrated. However, the water quality measurement device 100 may include a plurality of containing sections and be configured such that the measurement target liquid can be supplied from each containing section to the flow channel 130. The containing section 172 is a container in which water (pure water) used for cleaning or the like is contained. Each of the containing sections 174B, 174C, 174D, and 174E is a container in which a calibration liquid for zero calibration or span calibration is stored. The containing section 176A is a container for containing an acid (for example, sulfuric acid). The containing section 176B is a container for containing a base (for example, aqueous sodium hydroxide).

The flow channel 130 is a flow channel configured such that the measurement target liquid 190 flows from the containing section 178 to the sensor section 110 through the flow channel 130 and the measurement target liquid 190 is drained from the sensor section 110 through the flow channel 130. Of the flow channel 130, a flow channel portion disposed between an electromagnetic valve 140 and an electromagnetic valve 166 is a first flow channel 132. Of the flow channel 130, a flow channel portion disposed between the electromagnetic valve 166 and an electromagnetic valve 146 is a second flow channel 134. Of the flow channel 130, a flow channel portion disposed between the electromagnetic valve 140 and the containing section 178 is a third flow channel 136. Of the flow channel 130, a flow channel portion disposed on the downstream side of the electromagnetic valve 146 is a fourth flow channel 138.

The control section 120 is configured in the form of an information processing device and has a function for performing various types of controls. The control section 120 also has a function for obtaining various signals and a function for performing various types of information processing. The control section 120 controls operation of the drive section 115.

The drive section 115 is a device for producing drive in the flow channel 130 and for controlling flow of fluid in the flow channel 130. The drive section 115 can perform a drive operation of forcing the liquid to flow in the flow channel 130 and stopping the flow. The drive section 115 is controlled by the control section 120. The drive section 115 includes electromagnetic valves 140, 142A, 142B, 142C, 142D, 142E, 146, and 166. The drive section 115 also includes three-way valves 162 and 168. The drive section 115 includes pumps 150 and 154 as well. In the present configuration, the drive section 115 may correspond to one example of the supply section, or a portion that remains after exclusion of the electromagnetic valve 140 and the electromagnetic valve 146 from the drive section 115 may correspond to one example of the supply section. The supply section is a device for feeding the liquid to the flow channel 130. The supply section can perform, for example, an operation of feeding the measurement target liquid 190 into a closed space 135 (which will be described later) when the electromagnetic valve 140 is in an open state for opening the flow channel 130 and the electromagnetic valve 146 is in an open state for opening the flow channel 130.

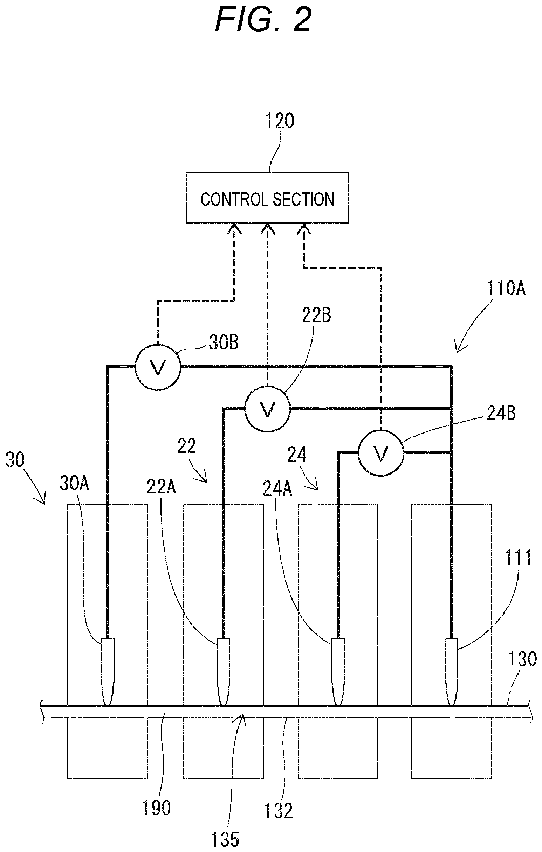

The electromagnetic valve 140 is an electromagnetic valve which opens and closes in accordance with the control performed by the control section 120. The electromagnetic valve 140 is switched between a state in which the electromagnetic valve 140 permits flow at a predetermined position of the flow channel 130 and a state in which the electromagnetic valve 140 does not permit the flow. The electromagnetic valve 140 corresponds to one example of the first opening-closing section and corresponds to one example of the first electromagnetic valve. The electromagnetic valve 140 opens and closes the flow channel 130 at a first position on the upstream side of the closed space 135 (FIG. 2), which will be described later. The electromagnetic valve 140 is switched between an open state (first open state) for opening the flow channel 130 at the first position and a closed state (second closed state) for closing the flow channel 130 at the first position. Each of the electromagnetic valves 142A, 142B, 142C, 142D, and 142E is a valve which is switched between a state in which the valve permits flow between a corresponding one of the containing sections 172, 174B, 174C, 174D, and 174E and the three-way valve 162 and a state in which the valve does not permit the flow . Notably, in the present specification, the upstream side in the flow channel 130 means the upstream side in a direction in which the measurement target liquid 190 is forced to flow, and the downstream side in the flow channel 130 means the downstream side in the direction in which the measurement target liquid 190 is forced to flow.

The electromagnetic valve 146 is an electromagnetic valve which opens and closes in accordance with the control performed by the control section 120. The electromagnetic valve 146 is switched between a state in which the electromagnetic valve 146 permits flow at a predetermined position of the flow channel 130 and a state in which the electromagnetic valve 146 does not permit the flow. The electromagnetic valve 146 corresponds to one example of the second opening-closing section and corresponds to one example of the second electromagnetic valve. The electromagnetic valve 146 opens and closes the flow channel 130 at a second position on the downstream side of the closed space 135 (FIG. 2), which will be described later. The electromagnetic valve 146 is switched between an open state (second open state) for opening the flow channel 130 at the second position and a closed state (second closed state) for closing the flow channel 130 at the second position. The second position is a position in the flow channel 130 at which the electromagnetic valve 146 is provided. When the electromagnetic valve 146 is in the open state (second open state), the electromagnetic valve 146 opens the flow channel 130, thereby permitting flow of liquid from the upstream side of the electromagnetic valve 146 toward the downstream side of the electromagnetic valve 146. When the electromagnetic valve 146 is in the closed state (second closed state), the electromagnetic valve 146 closes the flow channel 130, thereby interrupting flow of liquid from the upstream side of the electromagnetic valve 146 toward the downstream side of the electromagnetic valve 146.

The three-way valve 162 is controlled by the control section 120. The three-way valve 162 is a valve which can switch a source for feeding liquid to a portion of the flow channel 130 located on the downstream side of the three-way valve 162; i.e., can switch the source between the containing section 178 side and the side where the containing sections 172, 174B, 174C, 174D, and 174E are provided. When the three-way valve 162 is in a first changeover state, supply of liquid from the containing section 178 side to the sensor section 110 side is permitted, and supply of liquid from the side where the containing sections 172, 174B, 174C, 174D, and 174E are provided to the sensor section 110 side is interrupted. When the three-way valve 162 is in a second changeover state, supply of liquid from the side where the containing sections 172, 174B, 174C, 174D, and 174E are provided to the sensor section 110 side is permitted, and supply of liquid from the containing section 178 side to the sensor section 110 side is interrupted.

The electromagnetic valve 166 is controlled by the control section 120. The electromagnetic valve 166 is switched between an open state in which the electromagnetic valve 166 permits flow of liquid between the supply path 182 and the flow channel 130 and a closed state in which the electromagnetic valve 166 does not permit the flow. When the electromagnetic valve 166 is in the closed state, flow of liquid between the supply path 182 and the flow channel 130 is interrupted, whereby supply of an acid or a base from the supply path 182 side to the flow channel 130 is interrupted. When the electromagnetic valve 166 is in the open state, the supply of liquid from the supply path 182 side to the flow channel 130 side is permitted. A merging portion 130C is a portion where a supply path extending from the electromagnetic valve 166 side is connected to the flow channel 130 and where the liquid flowing from the electromagnetic valve 166 side can merge into the liquid flowing through the flow channel 130.

The three-way valve 168 is controlled by the control section 120. The three-way valve 168 is a valve which can switch a source for feeding liquid to the supply path 180; i.e., can switch the source between the containing section 176A side and the containing section 176B side. When the three-way valve 168 is in a first changeover state, the three-way valve 168 permits flow of liquid between the containing section 176A and the supply path 180 and interrupts flow of liquid between the containing section 176B and the supply path 180, thereby allowing supply of the acid contained in the containing section 176A to the supply path 180. When the three-way valve 168 is in a second changeover state, the three-way valve 168 permits- flow of liquid between the containing section 176B and the supply path 180 and interrupts flow of liquid between the containing section 176A and the supply path 180, thereby allowing supply of the base contained in the containing section 176B to the supply path 180.

The supply path 180 is a path through which the liquid supplied from the three-way valve 168 flows, and its one end is connected to the three-way valve 168. The other end of the supply path 180 is connected to the pump 154. The supply path 182 is a path for supplying to the electromagnetic valve 166 side the liquid supplied to the pump 154 side via the supply path 180.

The pump 150, which is a well known pump, is provided in the flow channel 130. The pump 150 has a function of feeding out the liquid in the flow channel 130 to the downstream side when the pump 150 is operating. Specifically, the pump 150 has a function of feeding out the liquid on the three-way valve 162 side to the sensor section 110 side when the pump 150 is operating. The pump 154, which is a well known pump, is provided between the supply path 180 and the supply path 182. The pump 154 has a function of feeding out the liquid in the flow channel 180 to the supply path 182 side (the electromagnetic valve 166 side) when the pump 154 is operating.

A flow-rate sensor 152 is a sensor for measuring the flow rate of the liquid flowing through the flow channel 130. The flow-rate sensor 152 detects the flow rate in the closed space 135, which will be described later, (specifically, the flow rate in the first flow channel 132). A flow-rate sensor 156 is a sensor for measuring the flow rate of the liquid flowing through the supply path 182.

Notably, although not illustrated, a filtration section for performing filtration treatment on the liquid flowing through the flow channel 130 may be provided in the flow channel 130. This filtration section may be configured to remove foreign substances from the liquid flowing through the flow channel 130.

The internal structure of the first sensor section 110A is schematically shown in FIG. 2.

Ions (target ions) whose concentrations are to be measured at the first sensor section 110A are determined in advance. The first sensor section 110A measures the concentrations of a plurality of types of target ions contained in the liquid flowing into the first sensor section 110A via the flow channel 130. In the case where the measurement target liquid 190 is supplied from the containing section 178 to the sensor section 110, "the liquid flowing into the sensor section 110" is the measurement target liquid 190. In the example of FIG. 1, the target ions whose concentrations are measured at the first sensor section 110A are calcium and magnesium ions. In the case where a calibration liquid is supplied from one of the containing sections 174B, 174C, 174D, and 174E to the sensor section 110, "the liquid flowing into the sensor section 110" is the calibration liquid.

As shown in FIG. 2, the first sensor section 110A includes a pH sensor 30, a calcium ion sensor 22, a magnesium ion sensor 24, and a reference electrode 111.

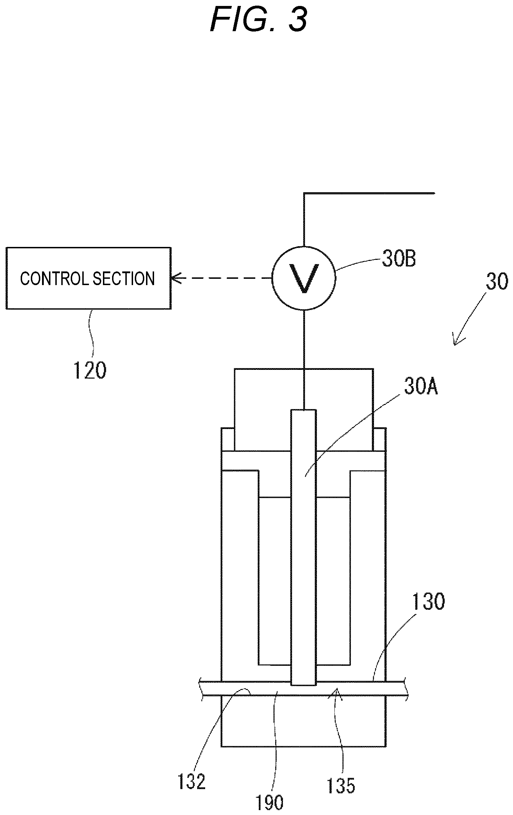

The pH sensor 30 corresponds to one example of the glass-electrode-type sensor. The pH sensor 30 operates in accordance with the same principle as a well known pH sensor. The pH sensor 30 is provided in the flow channel 130 to be located on the upstream side of the calcium ion sensor 22. More specifically, the pH sensor 30 is provided in the first flow channel 132 to be located on the upstream side of the calcium ion sensor 22. The pH sensor 30 includes an electrode portion 30A and a potentiometer 30B. The electrode portion 30A is a glass electrode. The pH sensor 30 has, for example, a configuration as shown in FIG. 3. The electrode portion 30A is exposed to a space in the first flow channel 132 (a portion of the closed space 135). In a state in which the liquid fills the closed space 135, the surface of the electrode portion 30A (the surface of the glass electrode) is immersed in liquid in the closed space 135. The closed space 135 is a space which is closed when the electromagnetic valves 140, 146, and 166 are brought into their closed state and the three-way valve 162 is brought into its first changeover state. Notably, when the electromagnetic valves 140 and 146 are brought into their open states, the closure of the closed space 135 is canceled, and the liquid is permitted to flow through the closed space 135. The closed space 135 is a common space in which a part of the electrode portion 30A of the pH sensor 30, a part of a responsive film portion of the calcium ion sensor 22, and a part of a responsive film portion of the magnesium ion sensor 24 are disposed. The closed space 135 contains the space in the first flow channel 132 and the space in the second flow channel 134.

When the measurement target liquid 190 fills the flow channel 130 (namely, the liquid fills the closed space 135), the surface of the electrode portion 30A of the pH sensor 30 (the surface of the glass electrode) is immersed in the measurement target liquid 190 and comes into contact with the measurement target liquid 190. At that time, the pH sensor 30 generates, at the electrode portion 30A, a potential corresponding to the pH of the measurement target liquid 190 in the vicinity of the electrode portion 30A. The potentiometer 30B measures the difference between the potential of the electrode portion 30A and the potential of the reference electrode 111 and outputs to the control section 120 a signal indicating the potential difference (a voltage signal corresponding to the pH of the measurement target liquid 190).

The reference electrode 111 shown in FIG. 2 is a well known reference electrode (comparative electrode) and generates a reference potential which serves as a reference for the electrode potentials of the pH sensor 30, the calcium ion sensor 22, the magnesium ion sensor 24, and the pH sensor 32.

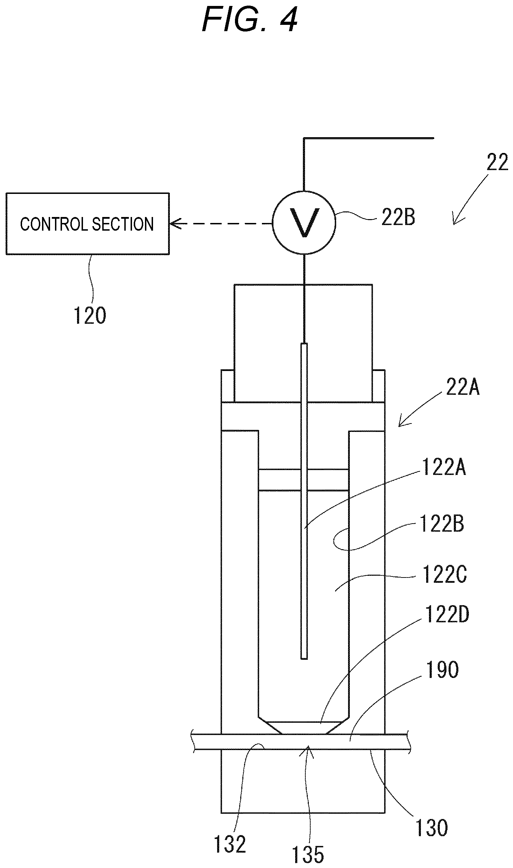

The calcium ion sensor 22 shown in FIG. 2 corresponds to one example of the ion sensor having a responsive film portion whose surface is disposed in the flow channel, and specifically, corresponds to one example of the liquid-membrane-type ion sensor. As shown in FIG. 2, the calcium ion sensor 22 is disposed in the flow channel 130 to be located on the downstream side of the pH sensor 30. The calcium ion sensor 22 includes an electrode portion 22A and a potentiometer 22B. The calcium ion sensor 22 is configured, for example, as shown in FIG. 4. The electrode portion 22A includes a responsive film portion (liquid membrane) 122D, an internal solution 122C, a containing portion 122B for containing the internal solution 122C, and an internal electrode 122A. The surface of the responsive film portion 122D of the electrode portion 22A is disposed in the flow channel. The responsive film portion 122D is a liquid-membrane-type ion responsive film on which an ionophore is supported.

The calcium ion sensor 22 operates in accordance with, for example, the same principle as a well known liquid-membrane-type calcium ion sensor. When the measurement target liquid 190 fills the flow channel 130 (namely, the liquid fills the closed space 135), the surface of the responsive film portion 122D is immersed in the measurement target liquid 190 and comes into contact with the measurement target liquid 190. At that time, the calcium ion sensor 22 generates, at the internal electrode 122A, a potential corresponding to the calcium ion concentration of the measurement target liquid 190 in the vicinity of the responsive film portion 122D, The potentiometer 22B measures the potential difference between the electrode potential of the internal electrode 122A and the electrode potential of the reference electrode 111 and outputs to the control section 120 a signal indicating the potential difference (a voltage signal indicating the potential difference corresponding to the calcium ion concentration).

The magnesium ion sensor 24 shown in FIG. 2 corresponds to one example of the ion sensor having a responsive film portion whose surface is disposed in the flow channel, and specifically, corresponds to one example of the liquid-membrane-type ion sensor. The magnesium ion sensor 24 is disposed in the flow channel 130 to be located on the downstream side of the calcium ion sensor 22. The magnesium ion sensor 24 includes an electrode portion 24A and a potentiometer 24B. The electrode portion 24A has the same configuration as the electrode portion 22A shown in FIG. 4. The electrode portion 24A also includes a responsive film portion which is the same as that of the electrode portion 22A, and the surface of the responsive film portion is disposed in the flow channel. The magnesium ion sensor 24 also operates in accordance with, for example, the same principle as a well known liquid-membrane-type magnesium ion sensor. When the measurement target liquid 190 fills the flow channel 130 (namely, the liquid fills the closed space 135), the magnesium ion sensor 24 generates, at its internal electrode, a potential corresponding to the magnesium ion concentration of the measurement target liquid 190 in the vicinity of the responsive film portion. The potentiometer 22B measures the potential difference between the electrode potential of the internal electrode and the electrode potential of the reference electrode 111 and outputs to the control section 120 a signal indicating the potential difference (a voltage signal indicating the potential difference corresponding to the magnesium ion concentration).

As described above, in the water quality measurement device 100, the surfaces of the responsive film portions of the calcium ion sensor 22 and the magnesium ion sensor 24 and the surface of the glass electrode of the pH sensor 30 are provided in the closed space 135 (common space) in the flow channel 130. In the water quality measurement device 100, when the electromagnetic valve 140 (the first opening-closing section) is in its closed state (first closed state), the closed space 135 is cut off from the space on the upstream side of the closed space 135 (specifically, the space in the third flow channel 136). Furthermore, in the water quality measurement device 100, when the electromagnetic valve 146 (the second opening-closing section) is in its closed state (second closed state), the closed space 135 is cut off from the space on the downstream side of the closed space 135 (specifically, the space in the fourth flow channel 138).

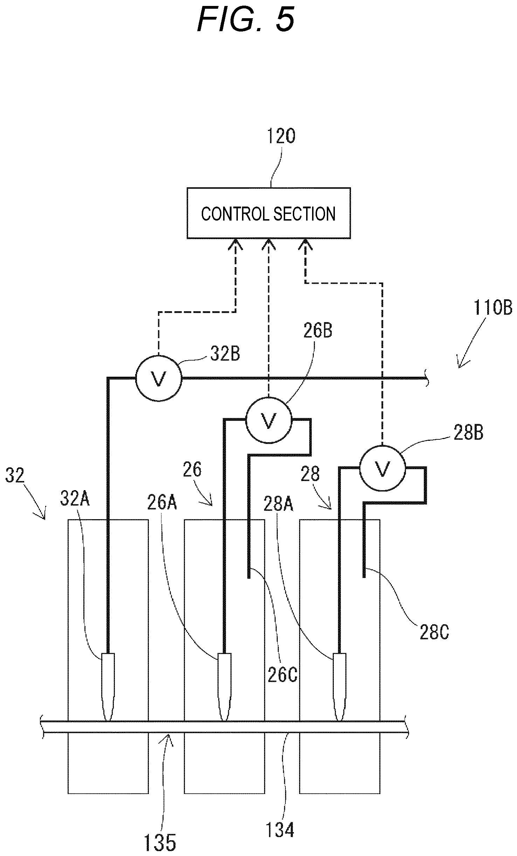

As shown in FIG. 5, the second sensor section 110B includes a pH sensor 32, an ammonia sensor 26, and a nitrous acid sensor 28.

The pH sensor 32 has the same configuration as the pH sensor 30 (FIG. 3) and functions in the same manner. When the measurement target liquid 190 fills the second flow channel 134, the pH sensor 32 generates, at the electrode portion 32A, a potential corresponding to the pH of the measurement target liquid 190. The potentiometer 32B measures the difference between the potential of the electrode portion 32A and the potential of the reference electrode 111 (FIG. 2) and outputs to the control section 120 a signal indicating the potential difference (a voltage signal corresponding to the pH of the measurement target liquid 190).

The nitrous acid sensor 28 measures the concentrations of nitrite ion or nitrous acid gas (measurement targets) contained in the measurement target liquid 190 after addition of an acid thereto by an acid addition operation, which will be described later. The nitrous acid sensor 28 is provided in the flow channel 130 to be located on the downstream side of the electromagnetic valve 166 (FIG. 1) (specifically, on the downstream side of the merging portion 130C). The three-way valve 168 and the supply path 182 shown in FIG. 1 serve as a path for adding an acid (for example, sulfuric acid) to the measurement target liquid 190. As a result of addition of the acid through this path, nitrite ion contained in the measurement target liquid 190 can be transformed into nitrous acid, which forms a gas. Specifically, the three-way valve 168 is switched such that the acid can be supplied from the containing section 176A, and the electromagnetic valve 166 is switched to its open state such that the acid can be supplied from the supply path 182 to the flow channel 130. When the pump 154 performs a flow causing operation, the acid is supplied to the flow channel 130 (specifically, the second flow channel 134). The supply of the acid is adjusted such that the pH of the measurement target liquid 190 after the supply (the measurement target liquid 190 on the downstream side of the electromagnetic valve 166 (specifically, on the downstream side of the merging portion 130C)) becomes 1.5 or lower.

The nitrous acid sensor 28 is configured in the form of a well known diaphragm-type ion sensor and includes an electrode portion 28A and a potentiometer 28B. The electrode portion 28A is configured in the form of a well known diaphragm-type ion electrode and includes an internal solution (electrolytic solution) in the electrode portion and an internal electrode (for example, a glass electrode). The electrode portion 28A is configured, for example, such that nitrous acid gas passes through its diaphragm and dissolves into the internal solution, and an electromotive force corresponding to the pH of the internal solution which changes due to the nitrous acid gas is generated between the internal electrode and the reference electrode. In the manner as described above, the electrode portion 28A can detect nitrous acid gas selectively. The electrode portion 28A is immersed in the liquid flowing through the flow channel 130 (specifically, the second flow channel 134) on the downstream side of the merging portion 130C. The electrode portion 28A generates an electrode potential corresponding to the concentration of nitrous acid gas contained in the acid-added measurement target liquid 190 (nitrous acid gas composed of nitrous acid gas that has been contained in the measurement target liquid 190 before passing through the merging portion 130C and nitrous acid gas formed from nitrite ion after the measurement target liquid 190 has passed through the merging portion 130C). The potentiometer 28B measures the difference between the electrode potential of the internal electrode of the electrode portion 28A and the electrode potential of the reference electrode and outputs to the control section 120 a signal indicating that potential difference (a signal indicating a potential difference corresponding to the concentration of nitrous acid gas). The reference electrode 28C of the nitrous acid sensor 28 is disposed in the internal solution.

The ammonia sensor 26 measures the concentrations of ammonium ion or ammonia (measurement targets) contained in the measurement target liquid 190 after addition of a base thereto by a base addition operation, which will be described later. The ammonia sensor 26 is provided in the flow channel 130 to be located on the downstream side of the electromagnetic valve 166 (FIG. 1) (specifically, on the downstream side of the merging portion 130C). The electromagnetic valve 166 and the supply path 182 shown in FIG. 1 also serve as a path for adding a base to the measurement target liquid 190. As a result of addition of the base through this path, ammonium ion contained in the measurement target liquid 190 can be transformed into ammonia gas. Specifically, the three-way valve 168 is switched such that the base can be supplied from the containing section 176B to the supply path 180, and the electromagnetic valve 166 is switched to its open state such that the liquid can be supplied from the supply path 182 to the flow channel 130. When the pump 154 performs a flow causing operation, the base is supplied to the flow channel 130 (specifically, the second flow channel 134). The supply of the base is adjusted, for example, such that the pH of the measurement target liquid 190 after the supply (the measurement target liquid 190 on the downstream side of the electromagnetic valve 166 (specifically, on the downstream side of the merging portion 130C)) becomes 11.5 or higher.

The ammonia sensor 26 is configured in the form of a well known diaphragm-type ion sensor and includes an electrode portion 26A and a potentiometer 26B. The electrode portion 26A is configured in the form of a well known diaphragm-type ion electrode and includes an internal solution (electrolytic solution) in the electrode portion and an internal electrode (for example, a glass electrode). The electrode portion 26A is configured, for example, such that ammonia gas passes through its diaphragm and dissolves into the internal solution, and an electromotive force corresponding to the pH of the internal solution which changes due to the ammonia gas is generated between the internal electrode and the reference electrode. In the manner as described above, the electrode portion 26A can detect ammonia gas selectively. The electrode portion 26A is immersed in the liquid flowing through the flow channel 130 (specifically, the second flow channel 134) on the downstream side of the merging portion 130C. The electrode portion 26A generates an electrode potential corresponding to the concentration of ammonia gas contained in the base-added measurement target liquid 190 (namely, ammonia gas composed of ammonia gas that has been contained in the measurement target liquid 190 before passing through the merging portion 130C and ammonia gas formed from ammonium ion upon passage of the merging portion 130C). The potentiometer 26B measures the difference between the electrode potential of the internal electrode of the electrode portion 26A and the electrode potential of the reference electrode and outputs to the control section 120 a signal indicating that potential difference (a signal indicating a potential difference corresponding to the concentration of ammonia gas). The reference electrode 26C of the ammonia sensor 26 is disposed in the internal solution.

### 2. Measurement control

The next description relates to a measurement control performed in the water quality measurement device 100. A storage method for the water quality measurement device 100 is realized by a measurement control, a cleaning control, a cancellation control (which will be described later), etc. which are performed by the water quality measurement device 100.

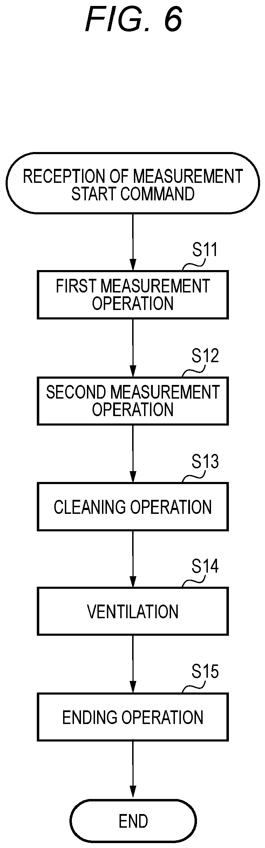

In the water quality measurement device 100 shown in FIG. 1, the control section 120 starts the measurement control show in FIG. 6 when a measurement start condition is satisfied. The measurement start condition may be, for example, that a predetermined measurement start operation is performed by an unillustrated operation device, or may be any of other measurement start conditions (for example, arrival of a scheduled measurement time set beforehand).

As shown in FIG. 6, the control section 120 shown in FIG. 1 performs a first measurement operation upon satisfaction of the above-described measurement start condition (step S11). The first measurement operation is an operation of measuring the pH, calcium ion, and magnesium ion of the measurement target liquid 190 by the first sensor section 110A while feeding the measurement target liquid 190 shown in FIG. 1 to the first flow channel 132 and measuring the pH and ammonia gas of the base-added measurement target liquid 190 by the second sensor section 110B while feeding the base-added measurement target liquid 190 to the second flow channel 134. In step S11 (FIG. 6), the control section 120 switches the electromagnetic valve 140 to its open state, switches the three-way valve 162 to its first changeover state, and activates the pump 150. As a result of these operations, the measurement target liquid 190 stored in the containing section 178 is supplied from the containing section 178 to the first flow channel 132. Notably, during the first measurement operation, supply of liquid from the containing sections 172, 174B, 174C, 174D, and 174E to the flow channel 130 is interrupted, and supply of liquid from the supply path 181 to the first flow channel 132 is interrupted.

When this first measurement operation is being performed, the pH sensor 30 measures the pH of the measurement target liquid 190 flowing through the first flow channel 132. The calcium ion sensor 22 measures the calcium ion concentration of the measurement target liquid 190 flowing through the first flow channel 132. The magnesium ion sensor 24 measures the magnesium ion concentration of the measurement target liquid 190 flowing through the first flow channel 132.

At the time of the first measurement operation, the control section 120 switches The electromagnetic valve 166 to its open state, thereby permitting flow of liquid between the supply path 182 and the flow channel 130 and permitting supply of liquid from the first sensor section 110A side to the second sensor section 110B side. Furthermore, the control section 120 switches the three-way valve 168 to its second changeover state, thereby permitting flow of liquid between the containing section 176B and the supply path 180 and activates the pump 154. As a result of these operations, the base stored in the containing section 176B is supplied to the second flow channel 134.

When this first measurement operation is being performed, the pH sensor 32 measures the pH of the measurement target liquid 190 flowing through the second flow channel 134. The ammonia sensor 26 measures ammonia gas contained in the measurement target liquid 190 flowing through the second flow channel 134.

After step S11 of FIG. 6, the control section 120 performs a second measurement operation in step S12. The second measurement operation is an operation of adding the acid to the measurement target liquid 190 shown in FIG. 1 and measuring the pH and nitrous acid gas of the acid-added measurement target liquid 190 by the second sensor section 110B while feeding the acid-added measurement target liquid 190 to the second flow channel 134. In step S12 (FIG. 6), the control section 120 switches the electromagnetic valve 140 to its open state (first open state), switches the three-way valve 162 to its first changeover state, and activates the pump 150. As a result of these operations, the measurement target liquid 190 stored in the containing section 178 is supplied from the containing section 178 to the first flow channel 132. At that time, supply of liquid from the containing sections 172, 174B, 174C, 174D, and 174E to the flow channel 130 is interrupted, and supply of liquid from the supply path 181 to the first flow channel 132 is interrupted. Furthermore, the control section 120 switches the electromagnetic valve 166 to its open state, thereby permitting flow of liquid between the supply path 182 and the flow channel 130 and permitting supply of liquid from the first sensor section 110A side to the second sensor section 110B side. Furthermore, the control section 120 switches the three-way valve 168 to its first changeover state, thereby permitting flow of liquid between the containing section 176A and the supply path 180 and activates the pump 154. As a result of these operations, the acid stored in the containing section 176A is supplied to the second flow channel 134.

When such second measurement operation is being performed, the pH sensor 32 measures the pH of the measurement target liquid 190 flowing through the second flow channel 134. The nitrous acid sensor 28 measures nitrous acid gas contained in the measurement target liquid 190 flowing through the second flow channel 134.

After step S12 of FIG. 6, the control section 120 performs the cleaning operation in step S13. The cleaning operation is an operation of permitting flow of water (pure water) from the containing section 172 to the flow channel 130, thereby performing cleaning. At the time of the cleaning operation, the control section 120 controls the pump 150, the electromagnetic valves 140, 146, and 166, the three-way valve 162, and the electromagnetic valves 142A to 142E in such a manner as to interrupt the supply of the measurement target liquid 190 and the calibration liquid, feed the water (pure water serving as cleaning water) in the containing section 172 to the first flow channel 132, and permit flow of liquid between the first flow channel 132 and the second flow channel 134. At that time, the control section 120 switches the electromagnetic valve 140 to its interruption state, switches the electromagnetic valve 146 to its open state, switches the electromagnetic valve 142Ato its open state, and switches the electromagnetic valves 142B to 142E to their interruption states. Furthermore, the control section 120 switches the three-way valve 162 to its second changeover state and switches the electromagnetic valve 166 to its closed state. The control section 120 activates the pump 150 in this state, thereby feeding the water (pure water serving as cleaning water) in the containing section 172 to the first flow channel 132. As a result of such cleaning operation, the first flow channel 132 and the second flow channel 134 are flushed with water.

In this example, the electromagnetic valve 140, the electromagnetic valve 142A, the three-way valve 162, and the pump 150 correspond to one example of the cleaning section and can perform the cleaning operation of feeding the cleaning water to the closed space 135. Also, in this example, the case where the condition for starting step S13 is satisfied (specifically, the case where the second measurement operation of step S12 ends) corresponds to one example of "when the storage condition is satisfied," and the control section 120 causes the cleaning section to perform the above-described cleaning operation when the storage condition is satisfied.

After step S13 of FIG. 6, in step S14, the control section 120 causes the drive section 115 to perform a ventilation operation. The ventilation operation is an operation of draining liquid from the closed space 135 and feeding air into the closed space 135; specifically, an operation of draining liquid from the first flow channel 132 and the second flow channel 134 and feeding air into the first flow channel 132 and the second flow channel 134. When the control section 120 performs the ventilation operation in step S14, the control section 120 switches the electromagnetic valve 140 to its closed state, thereby stopping supply of the measurement target liquid 190, and switches the three-way valve 162 to its first changeover state, thereby stopping supply of liquid from the containing sections 172 and 174B to 174E. Furthermore, the control section 120 switches the electromagnetic valve 166 to its closed state, switches the electromagnetic valve 146 to its open state (second open state), and activates the pump 150. As a result of this operation, the liquid remaining in the flow channel 130 between the electromagnetic valve 140 and the electromagnetic valve 146 is drained from the fourth flow channel 138. At that time, air (atmospheric air) is fed into the flow channel 130 from an unillustrated air vent (for example, an air vent provided between the electromagnetic valve 140 and the three-way valve 162. This air vent may have any configuration so long as the flow of liquid to the outside is interrupted at ordinary time and inflow of air (atmospheric air) is permitted at the time of ventilation operation. For example, the air vent may be opened and closed by an electromagnetic valve or may be provided with a check valve.

The control section 120 performs such ventilation operation until a predetermined ventilation stop condition is satisfied. The ventilation stop condition may be that the flow rate detected by the flow-rate sensor 152 has become less than a threshold value, that the flow rate detected by the flow-rate sensor 152 has been maintained at a flow rate less than the threshold value for a certain period of time, or that a predetermined period of time has elapsed after the flow rate detected by the flow-rate sensor 152 had become less than the threshold value. Alternatively, the ventilation stop condition may be that a predetermined period of time has elapsed after the start of the ventilation operation of step S14. In any example, it is sufficient that, when the ventilation stop condition is satisfied, a "state (wet state) in which the surface of the responsive film portion is not immersed in liquid and liquid is present in the closed space 135" is established. Namely, in any case, the above-described threshold value, the above-described certain period of time, the above-described predetermined period of time, and the above-described certain elapsed time are set in consideration of the characteristics of the device such that the above-described wet state is established without fail when the ventilation stop condition is satisfied. The "state in which liquid is present in the closed space 135" mentioned here; namely, the wet state at the point in time when formation of the closed space 135 starts, does not mean a state in which liquid is present in an amount that does not influence the suppression of humidity drop in the closed space 135 (for example, a state in which only one or two droplets of liquid are present in the closed space 135). This "state in which liquid is present in the closed space 135" means a state in which liquid is present in an amount that contributes to the suppression of humidity drop in the closed space 135. The state in which liquid is present in the closed space 135 may be a state in which a dozen or several tens of droplets of liquid are present in the closed space 135 or a state in which a larger number of droplets of liquid are present in the closed space 135, so long as the liquid is present in an amount that contributes to the suppression of humidity drop in the closed space 135. Also, the state in which liquid is present in the closed space 135 may be a state in which liquid is continuously present over a certain length or more in a region extending over the entire length or a portion of the closed space 135, or a state in which liquid is not continuously present over the entire length of the closed space 135 and droplets are present sparsely, so long as the liquid is present in an amount that contributes to the suppression of humidity drop in the closed space 135.

As described above, in the above-described "case where the storage condition is satisfied," after the above-described cleaning operation (step S13), the control section 120 causes the drive section 115 to perform the ventilation operation (the operation of draining liquid from the closed space 135 and the operation of feeding air into the closed space 135). This ventilation operation is an operation of bringing the interior of the closed space 135 into the above-described wet state.

After step S14 of FIG. 6, the control section 120 performs an ending operation in step S15. The ending operation is an operation of switching the electromagnetic valve 140 (the first opening-closing section) to its closed state (the first closed state) and switching the electromagnetic valve 146 (the second opening-closing section) to its closed state (the second closed state) in the above-described wet state. Notably, the ending operation is also an operation of maintaining the three-way valve 162 in its first changeover state and maintaining the electromagnetic valve 166 in its closed state. As a result of such operation, the closed space 135 (common space) becomes a confined space, flow of liquid between the closed space 135 and the space on the upstream side of the closed space 135 (the upstream side of the electromagnetic valve 140) is interrupted, and flow of liquid between the closed space 135 and the space on the downstream side of the closed space 135 (the downstream side of the electromagnetic valve 146) is interrupted. Therefore, so long as such a confined state is maintained, the interior of the closed space 135 is stably maintained in the above-described wet state until the closed state (the first closed state) of the electromagnetic valve 140 (the first opening-closing section) or the closed state (the second closed state) of the electromagnetic valve 146 (the second opening-closing section) is cancelled.

### 3. Cleaning control

The next description relates to a cleaning control performed in the water quality measurement device 100.

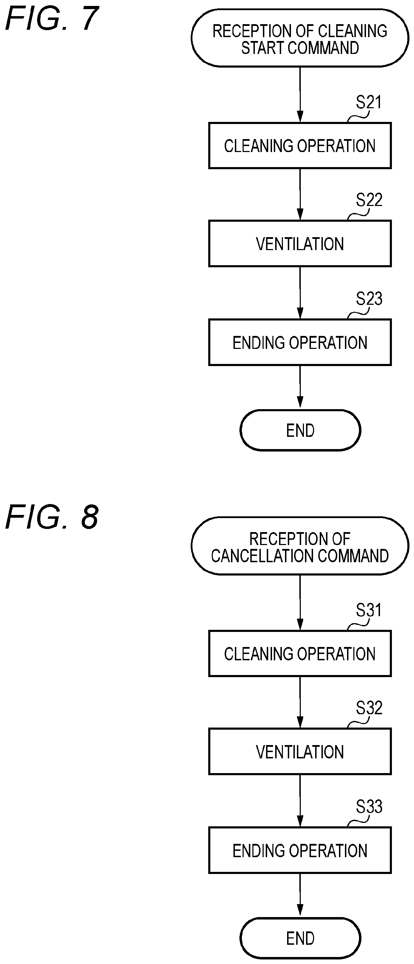

In the water quality measurement device 100 shown in FIG. 1, the control section 120 starts the cleaning control show in FIG. 7 when a cleaning start condition is satisfied. The cleaning start condition may be, for example, that a predetermined cleaning start operation is performed by an unillustrated operation device, or may be any of other cleaning start conditions (for example, arrival of a scheduled cleaning time set beforehand).

When the control section 120 starts the cleaning control of FIG. 7, the control section 120 first performs the cleaning operation in step S21. The cleaning operation of step S21 is the same as the cleaning operation of step S13 (FIG. 6) and an operation which can supply cleaning water to the closed space 135. In this example, the case where the above-described cleaning start condition is satisfied corresponds to one example of "when the storage condition is satisfied," and the control section 120 causes the cleaning section to perform the cleaning operation upon satisfaction of the storage condition.

After step S21 of FIG. 7, the control section 120 causes the drive section 115 to perform a ventilation operation in step S22. The ventilation operation of step S22 is the same operation as the ventilation operation of step S14 (FIG. 6) and includes the operation of draining liquid from the closed space 135 and the operation of feeding air into the closed space 135. The control section 120 performs such ventilation operation until a predetermined ventilation stop condition is satisfied. The ventilation stop condition is the same as that for the ventilation operation of step S14 (FIG. 6).

After step S22 of FIG. 7, the control section 120 performs an ending operation in step S23. The ending operation of step S23 is the same as the ending operation of step S15 (FIG. 6) and is an operation of switching the electromagnetic valve 140 (the first opening-closing section) to its closed state (the first closed state) and switching the electromagnetic valve 146 (the second opening-closing section) to its closed state (the second closed state) in the above-described wet state.

The water quality measurement device 100 can stably maintain the closed space 135 in the above-described wet state after such cleaning control.

### 4. Cancellation control

The next description relates to a cancellation control performed in the water quality measurement device 100.

In the water quality measurement device 100 shown in FIG. 1, the control section 120 starts the cancellation control show in FIG. 8 when a cancellation condition is satisfied. The cancellation condition may be, for example, that a predetermined cancellation operation is performed by an unillustrated operation device, or may be any of other cancellation conditions. The cancellation control of FIG. 8 is a control which can be executed in place of the measurement control of FIG. 6, for example, when the above-described cancellation condition is satisfied during execution of the measurement control of FIG. 6.

When the control section 120 starts the cancellation control of FIG. 8, the control section 120 first performs the cleaning operation in step S31. The cleaning operation of step S31 is the same as the cleaning operation of step S13 (FIG. 6) and the cleaning operation of step S21 (FIG. 7) and an operation which can supply cleaning water to the closed space 135. In this example, the case where the above-described cancellation condition is satisfied corresponds to one example of "when the storage condition is satisfied," and the control section 120 causes the cleaning section to perform the cleaning operation upon satisfaction of the storage condition.

After step S31 of FIG. 8, the control section 120 causes the drive section 115 to perform a ventilation operation in step S32. The ventilation operation of step S32 is the same operation as the ventilation operation of step S14 (FIG. 6) and the ventilation operation of step S22 (FIG. 7) and includes the operation of draining liquid from the closed space 135 and the operation of feeding air into the closed space 135. The control section 120 performs such ventilation operation until a predetermined ventilation stop condition is satisfied. The ventilation stop condition is the same as that for the ventilation operation of step S14 (FIG. 6).

After step S32 of FIG. 8, the control section 120 performs an ending operation in step S33. The ending operation of step S33 is the same as the ending operation of step S15 (FIG. 6) and the ending operation of step S23 (FIG. 7) and is an operation of switching the electromagnetic valve 140 (the first opening-closing section) to its closed state (the first closed state) and switching the electromagnetic valve 146 (the second opening-closing section) to its closed state (the second closed state) in the above-described wet state.

The water quality measurement device 100 can stably maintain the closed space 135 in the above-described wet state after such cancellation control.

### 5. Examples of effects

The next description relates to examples of effects of the water quality measurement device 100.

When the storage condition is satisfied, the water quality measurement device 100 can bring the interior of the closed space 135 into a wet state and close the opposite ends of the closed space 135 in the above-described wet state. The above-described wet state is a state in which the surface of the responsive film portion is not immersed in liquid and liquid is present in the closed space 135. Therefore, outflow of water from the closed space 135 and inflow of water into the closed space 135 are prevented after their opposite ends have been closed, whereby the above-described wet state is maintained. Namely, during a storage period after the opposite ends have been closed in the above-described wet state, the water quality measurement device 100 can maintain the state in which the surface of the responsive film portion is not immersed in liquid, thereby preventing malfunctions that would otherwise occur when the calcium ion sensor 22 and the magnesium ion sensor 24 are stored with their responsive film portions continuously immersed in liquid. Also, during the storage period after the opposite ends have been closed in the above-described wet state, since the water quality measurement device 100 can maintain the state in which liquid is present in the closed space 135, a drop in humidity can be suppressed, whereby drying of the surface of the glass electrode of the pH sensor 30 can be prevented.

Also, the water quality measurement device 100 can automatically perform the operation of producing the above-described wet state through own control when the storage condition is satisfied.

In the water quality measurement device 100, the electromagnetic valve 140 (the first opening-closing section) is a first electromagnetic valve which opens and closes the flow channel 130 on the upstream side of the closed space 135, and the electromagnetic valve 146 (the second opening-closing section) is a second electromagnetic valve which opens and closes the flow channel 130 on the downstream side of the closed space 135. By means of electromagnetic valves, this water quality measurement device 100 can reliably open and close the channel 130, at desired timings, at a first position on the upstream side of the closed space 135 and a second position on the downstream side of the closed space 135. Therefore, during the storage period after the opposite ends of the closed space 135 have been closed in the above-described wet state, the closed space 135 is more likely to be maintained in a state similar to the state before the closure.

In the water quality measurement device 100, when the storage condition is satisfied, the control section 120 causes the drive section 115 to perform the operation of draining liquid from the closed space 135 and the operation of introducing air into the closed space 135. In the water quality measurement device 100, during the storage period after the opposite ends have been closed in the above-described wet state, the surface of each responsive film portion is more likely to be maintained in a state in which the surface is exposed to air within the closed space 135. Therefore, this water quality measurement device 100 can reliably prevent malfunctions that would otherwise occur when the calcium ion sensor 22 and the magnesium ion sensor 24 are stored with their responsive film portions continuously immersed in liquid. Also, this water quality measurement device 100 can easily produce the above-described wet state (the state in which the surface of each responsive film portion is not immersed in liquid) through drainage of liquid from the closed space 135 and introduction of air into the closed space 135.

The above-described wet state may be a state in which the flow rate in the closed space 135 detected by the flow-rate sensor 152 becomes less than a predetermined value when the drive section 115 is performing the operation of draining liquid from the closed space 135 and feeding air into the closed space 135. This water quality measurement device 100 can quantitatively determine, on the basis of the "flow rate in the closed space 135," whether or not the state in which the surface of each responsive film portion is not immersed in liquid is produced.

When the storage condition is satisfied, the water quality measurement device 100 can perform an operation of cleaning the closed space 135 with cleaning water and close the opposite ends of the closed space 135 in the above-described wet state after such cleaning operation. Therefore, during the storage period after the opposite ends of the closed space 135 have been closed, this water quality measurement device 100 can store well the ion sensors having respective responsive film portions whose surfaces are disposed in the flow channel and the sensor using a glass electrode, while maintaining the interior of the closed space 135 in a cleaner state.

### <Other embodiments>

The present invention is not limited to the embodiment described by the above description with reference to the drawings. For example, the features of the above-described embodiment and embodiments which will be described below can be combined in any manner so long as no contradiction occurs. Also, any of the features of the above-described embodiment and embodiments which will be described below may be omitted unless explicitly stated as essential. Moreover, the above-described embodiment may be modified as follows.

In the above-described embodiment, the water quality measurement device 100 itself includes the control section. However, it is not required for the water quality measurement device to include the control section. For example, a control section which can perform the same operation as the above-described control section 120 may be provided separately from the water quality measurement device, and the water quality measurement device may be configured to be controlled by this control section. In the case where the control section is provided separately from the water quality measurement device as described above, this control section may be integrally incorporated into a portion or the entirety of the water quality measurement device, or may be provided at a location remote from the water quality measurement device and configured to be capable of remotely controlling the water quality measurement device.

The storage method for the water quality measurement device may be such that the first opening-closing section and the second opening-closing section are not switched under the control of the control section. For example, when the storage condition is satisfied, the first opening-closing section and the second opening-closing section may be brought into the first closed state and the second closed state, respectively, by an operator's manual operation.

In the above-described embodiment, a pH sensor is shown as an example of the glass-electrode-type sensor. However, the glass-electrode-type sensor is not limited to the pH sensor. No limitation is imposed on the glass-electrode-type sensor so long as the sensor performs measurement with its glass electrode immersed in a measurement target liquid, and the glass-electrode-type sensor may be a conductivity sensor, an ORP sensor (redox potential sensor), or the like.

In the above-described embodiment, the second opening-closing section is configured by the electromagnetic valve 146. However, the second opening-closing section may have any configuration so long as the second opening-closing section can open and close the flow channel. For example, the second opening-closing section may be configured in such a manner that a liquid tank is provided at an end portion of the flow channel 130 and the level of liquid in this liquid tank is changed between a level at which the end portion of the flow channel 130 is closed by the liquid and a level at which the end portion of the flow channel 130 is not closed by the liquid. The change in the level can be controlled through supply of the liquid to the liquid tank and discharge of the liquid from the liquid tank. Alternatively, an accumulation section may be provided in the flow channel 130 to be located on the downstream side of the second sensor section 110B. The liquid accumulates in the accumulation section so that the liquid can close the flow channel. In this case, a state in which the liquid has accumulated in the accumulation section and does not flow corresponds to the second closed state, and a state in which the liquid flows such that the liquid in the accumulation section is forced to flow corresponds to the second open state.

In the above-described embodiment, the electromagnetic valve 146 is one example of the second opening-closing section. However, an electromagnetic valve may be provided between the first flow channel 132 and the second flow channel and may function as the second opening-closing section by operating in such a manner as to establish communication between the space within the first flow channel 132 and the space on the downstream side of the first flow channel 132 or interrupt the communication.

In the above-described embodiment, "water used for product growth management" is shown as an example of the measurement target liquid, which is the measurement target of the water quality measurement device 100. However, its specific example is not limited to the above-described example. For example, a specific example of the "product growth management" may be "growth management in fisheries," "growth management in agriculture," "growth management in the livestock industry," "growth management in forestry," or the like. Alternatively, the "product growth management" may be growth management in other primary industries.

A specific example of the measurement target liquid, which is the measurement target of the water quality measurement device 100, may be water used for "aquaculture management of an aquatic organism." In this case, an example of the "water used for "aquaculture management of an aquatic organism" is breeding water used for breeding the aquatic organism. Also, an example of the "aquatic organism" is a shellfish such as prawn. However, the aquatic organism is not limited to this example and may be an organism (other than prawn) of *Decapoda, Malacostraca, Crustacea,* or *Arthropoda.* Alternatively, the aquatic organism may be fishes such as sea bream, shellfishes such as scallop, or seaweeds such as *Wakame* seaweed.

Notably, the embodiments disclosed this time should be considered to be illustrative and not to be restrictive in all aspects. The scope of the present invention is not limited to the embodiments disclosed this time, and it is intended that the present invention encompasses all modifications within the range shown by the claims and the range of equivalents of the claims.

### REFERENCE SIGNS LIST

22: calcium ion sensor (ion sensor)
24: magnesium ion sensor (ion sensor)
30: pH sensor (glass-electrode-type sensor)
30A: electrode portion (glass electrode)
100: water quality measurement device
115: drive section (supply section)
120: control section
122D: responsive film portion
130: flow channel
135: closed space
140: electromagnetic valve (first opening-closing section, first electromagnetic valve, cleaning section)
142A: electromagnetic valve (cleaning section)
150: pump (cleaning section)
152: flow-rate sensor
146: electromagnetic valve (second opening-closing section, second electromagnetic valve)
162: three-way valve (cleaning section)
190: measurement target liquid

## Claims

1. A water quality measurement device having a flow channel through which a measurement target liquid is forced to flow, including an ion sensor which has a responsive film portion and in which a surface of the responsive film portion is disposed in the flow channel, and measuring the quality of the measurement target liquid, the water quality measurement device comprising:
a glass-electrode-type sensor which has a glass electrode and in which a surface of the glass electrode is disposed in the flow channel;
a drive section for controlling a flow of a fluid in the flow channel;
a first opening-closing section which is provided at a first position and is switched between a first open state for opening the flow channel and a first closed state for closing the flow channel, the first position being located on an upstream side of the ion sensor and the glass-electrode-type sensor in a direction in which the measurement target liquid is forced to flow;
a second opening-closing section which is provided at a second position and is switched between a second open state for opening the flow channel and a second closed state for closing the flow channel, the second position being located on a downstream side of the ion sensor and the glass-electrode-type sensor in the direction in which the measurement target liquid is forced to flow; and
a supply section for supplying a liquid to the flow channel,
wherein, when a storage condition is satisfied, the first opening-closing section is switched to the first closed state and the second opening-closing section is switched to the second closed state, thereby producing a state in which the surface of the responsive film portion and the surface of the glass electrode are present in a common closed space, whereby the closed space is put in a wet state without the surface of the responsive film portion being immersed in the liquid.

2. A water quality measurement device according to claim 1, further comprising a control section for controlling operations of the drive section, the first opening-closing section, the second opening-closing section, and the supply section,
wherein, when the storage condition is satisfied, the control section causes the drive section to perform an operation of producing the wet state.

3. A water quality measurement device according to claim 2, wherein the first opening-closing section is a first electromagnetic valve for opening and closing the flow channel on the upstream side of the closed space, and the second opening-closing section is a second electromagnetic valve for opening and closing the flow channel on the downstream side of the closed space.

4. A water quality measurement device according to claim 2 or 3, wherein, when the storage condition is satisfied, the control section causes the drive section to perform an operation of draining the liquid from the closed space and an operation of feeding air into the closed space.

5. A water quality measurement device according to claim 4, further comprising a flow-rate sensor which can detect the flow rate of the liquid flowing through the closed space,
wherein the wet state is a state in which the flow rate in the closed space detected by the flow-rate sensor has become less than a predetermined value when the drive section is performing the operation of draining the liquid from the closed space and the operation of feeding air into the closed space.

6. A water quality measurement device according to any one of claims 2 to 5, wherein the supply section includes a cleaning section which performs a cleaning operation of feeding cleaning water to the closed space, and
wherein, when the storage condition is satisfied, the control section causes the cleaning section to perform the cleaning operation and causes the drive section to perform the operation of producing the wet state after the cleaning operation.

7. A storage method for a water quality measurement device having a flow channel through which a measurement target liquid is forced to flow, including an ion sensor which has a responsive film portion and in which a surface of the responsive film portion is disposed in the flow channel, and measuring the quality of the measurement target liquid, the water quality measurement device comprising:
a glass-electrode-type sensor which has a glass electrode and in which a surface of the glass electrode is disposed in the flow channel;
a drive section for controlling a flow of a fluid in the flow channel;
a first opening-closing section which is provided at a first position and is switched between a first open state for opening the flow channel and a first closed state for closing the flow channel, the first position being located on an upstream side of the ion sensor and the glass-electrode-type sensor in a direction in which the measurement target liquid is forced to flow;
a second opening-closing section which is provided at a second position and is switched between a second open state for opening the flow channel and a second closed state for closing the flow channel, the second position being located on a downstream side of the ion sensor and the glass-electrode-type sensor in the direction in which the measurement target liquid is forced to flow; and
a supply section for supplying a liquid to the flow channel,
wherein, when a storage condition is satisfied, the storage method switches the first opening-closing section to the first closed state and the second opening-closing section to the second closed state, thereby producing a state in which the surface of the responsive film portion and the surface of the glass electrode are present in a common closed space, whereby the closed space is put in a wet state without the surface of the responsive film portion being immersed in the liquid.

8. A storage method for a water quality measurement device according to claim 7, wherein the water quality measurement device includes a control section for controlling operations of the drive section, the first opening-closing section, the second opening-closing section, and the supply section,
wherein, when the storage condition is satisfied, the control section causes the drive section to perform an operation of producing the wet state.

9. A storage method for a water quality measurement device according to claim 8, wherein the first opening-closing section is a first electromagnetic valve for opening and closing the flow channel on the upstream side of the closed space, and the second opening-closing section is a second electromagnetic valve for opening and closing the flow channel on the downstream side of the closed space.

10. A storage method for a water quality measurement device according to claim 8 or 9, wherein, when the storage condition is satisfied, the control section causes the drive section to perform an operation of draining the liquid from the closed space and an operation of feeding air into the closed space.

11. A storage method for a water quality measurement device according to claim 10, wherein the water quality measurement device includes a flow-rate sensor which can detect the flow rate of the liquid flowing through the closed space, and
wherein the wet state is a state in which the flow rate in the closed space detected by the flow-rate sensor has become less than a predetermined value when the drive section is performing the operation of draining the liquid from the closed space and the operation of feeding air into the closed space.

12. A storage method for a water quality measurement device according to any one of claims 8 to 11, wherein the supply section includes a cleaning section which performs a cleaning operation of feeding cleaning water to the closed space, and
wherein, when the storage condition is satisfied, the control section causes the cleaning section to perform the cleaning operation and causes the drive section to perform the operation of producing the wet state after the cleaning operation.
